# EUROPEAN PATENT SPECIFICATION

(11) **EP 3 057 548 B1**
(45) Date of publication and mention of the grant of the patent: **28.08.2019**
(21) Application number: 14708934.6
(22) Date of filing: 27.01.2014
(51) Int. Cl.: A61F 7/00, H05B 1/02, A61F 7/02

(54) **ELECTRICALLY HEATED BLANKET WITH SLEEP DEPTH DEPENDENT CONTROL FOR WEIGHT LOSS**
ELEKTRISCH BEHEIZTE DECKE MIT SCHLAFTIEFENABHÄNGIGER GEWICHTSVERLUSTSTEUERUNG
COUVERTURE CHAUFFANTE ÉLECTRIQUE DISPOSANT D'UN RÉGLAGE DÉPENDANT DE LA PROFONDEUR DU SOMMEIL POUR PERDRE DU POIDS

(30) Priority: 18.10.2013 DE 202013009227 U
(43) Date of publication of application: 24.08.2016
(73) Proprietor: Fuhs, Manfred, 53474 Bad Neuenahr (DE)
(72) Inventor: Fuhs, Manfred, 53474 Bad Neuenahr (DE)
(74) Representative: Dieckhoff, Beate
(86) International application number: PCT/IB2014/000085
(87) International publication number: WO 2015/056057

(56) References cited:
- WO-A1-2013/061185
- DE-U1-202013 009 227
- DE-U1-202013 009 227

## Description

### FIELD OF THE INVENTION

The invention relates to a bed cover, more particularly to an electric blanket, which, however barely isolates, but only temporarily produces electrical warming.

### BACKGROUND OF THE INVENTION - PROBLEM TO BE SOLVED

The known physiological effect that light clothing and some shivering increase calorie consumption and thus helps in weight loss, has already led to many proposed concepts in diet, light clothing and to strive for more physical action.

However, the physiological effects causing chills with small, involuntary contractions of sub-cutanoeus muscles is discomforting to most obesed persons and is therefore avoided.

Additionally, there is the old superstition that colds were caused by temporal hypothermia - even though catching a cold is known to be caused by infections.

Therefore, such recipes have rarely been complied with.

### Prior Art

A technological solution to this problem is suggested in patent applications of Li Jun in CN2870640 (Y) Deshui Li with CN201888580 (U), as well as Hanschke in DE 10 2010 033 971 A1, which propose a blanket with circulating coolant, while several other innovations suggest the injection of cooling (or, for other purposes warming) air or transfer agents into bed covers, as proposed in DE 692 12 211 T2.

It is important to note however, that blankets with circulation of coolants inevitably must be rather stiff and uncomfortable and would be severely impaired in their function by inevitable kinks.
Moreover, the system requires high technical complexity for waterproofing or airtightness and maintaining circulation.
In addition, application is bound to high self-discipline, since the blankets already spread chill before falling asleep.
WO 2013/061185 A1 describes methods for sleep stage classification whereby sleep phases are determined by recording brain activity of a person over time with electrodes. This method can be used in a sleep stage classification system, and can comprise at least one switching or control means for a periphery device. This periphery device can be a heating blanket, for example.

### OBJECT OF THE INVENTION

It is therefore an object of the present invention, to develop a system for high user compliance.

This is achieved with only chilling the body within deep sleep phases and with a design, that is inexpensive and less intricate to use. The electrically heated blanket according to the invention has a sleep depth-dependent control for weight loss and no significant thermal insulation effect, wherein the heating of the blanket is controlled in response to sleep phases. These sleep phases are determined by motion sensors in the blanket, connected to a recording logic, and the heating of the blanket is reduced or turned off in deep sleep phases which are characterized by lack of movement.

### INVENTIVE STEP

The inventive step therefore is to control the heating with an analysis of the state of sleep, which is expediently done by inserting sensors into the blanket and to refrain from any active cooling, but to design the blanket so that it isolates the body heat as little as possible in its passive state and rises temperature only through the electric heating function - which can easily be controlled.

### TECHNO-MEDICAL BASIS

The invention is defined in appended independent claim 1, preferred embodiments are described in the dependent claims.

The sleep cycle comprises a change from deep sleep to light sleep with brief almost-awake moments. In this phase, possible cooling is perceived and would lead to awakening.

The average adult has about 4-5 full sleep cycles within 8 hours. Each cycle lasts between 90 and 110 minutes, and has 5 different phases, which vary individually and on external influences.

Phase 1 marks falling asleep, in phases 2 to 4 of the sleep grows deeper for the so-called delta sleep status, during which cooling causes no reduction in the depth of sleep or would provoke waking up.

The 5th Phase is called REM phase. This is the dream phase of accelerated respiration, increased brain activity and rapid eye movements under the eyelid (hence REM is the acronym for Rapid Eye Movement).

At this stage cooling would result in impairment of the sleep quality and therefore has to be avoided.

With the aid of an acceleration sensor, the short light-sleep phases (particularly the critical REM-phases) can be determined and largely predicted by the sequence analysis, so that the blanket can be heated up again before such a critical moment occurs.

### DESCRIPTION OF THE INVENTION

The blanket consists of a heating fabric as in conventional electric blankets, only that it is covered on both sides with lattice or heat-permeable fabric.

A few three-dimensional position sensors, in a one-chip design are integrated and report to an analytic electronic device.

The occurrence of position changes, which are registered this way, build the parameters for the identification of sleep phases, which may be assigned as to common model and on this basis be continuously predicted, however improved with increasing self-learning of the system on basis of evolution of registered data.

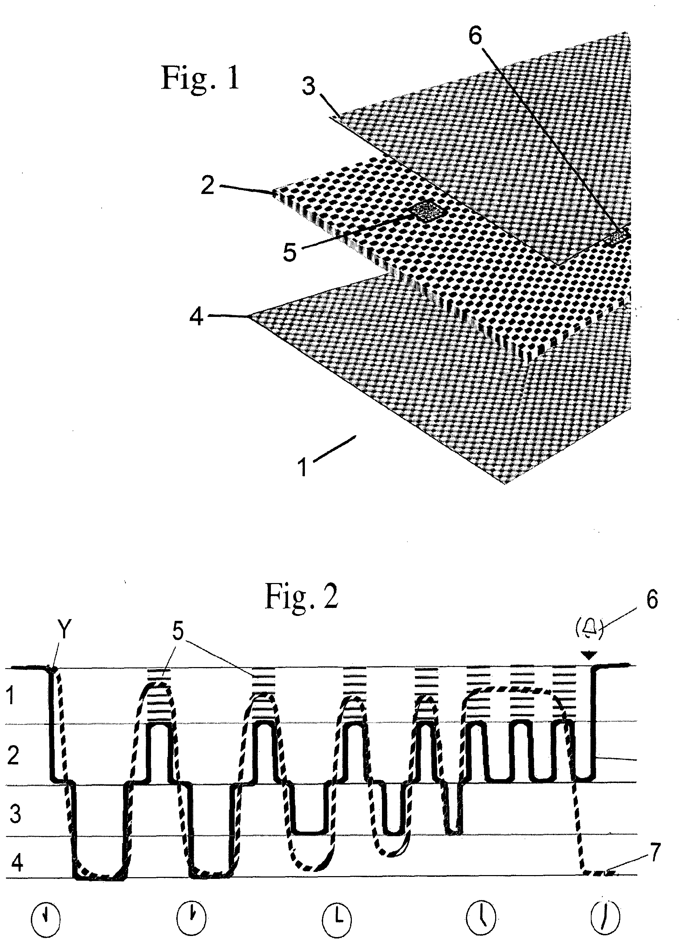

## Claims

1. Electrically heated blanket (1),
**characterized in that**
the blanket (1) has a sleep depth-dependent control for weight loss and no significant thermal insulation effect, wherein the heating of the blanket (1) is controlled in response to sleep phases and the sleep phases are determined by motion sensors (5,6) in the blanket (1), connected to a recording logic, and the heating of the blanket (1) is reduced or turned off in deep sleep phases which are **characterized by** lack of movement.

2. Electrically heated blanket as to claim 1, wherein coming sleep phases are predicted by evaluation of registered prior devolutions.

3. Electrically heated blanket as to claim 1, wherein renewed pressure on a start button prior to passage of an ordinary sweep is registered as incident due to possible temperature undercut.

4. Electrically heated blanket as to one or more of the preceding claims, wherein the switch-off of the heating is also used for - or at least supporting - waking up of the user at reveille time.

## Patentansprüche

1. Elektrisch heizbare Decke (1),
**dadurch gekennzeichnet, dass** die Decke (1) eine Schlaftiefenabhängige Steuerung zur Gewichtsreduktion und keine nennenswerte thermische Isolationswirkung aufweist, wobei die Beheizung der Decke (1) in Abhängigkeit von ermittelten Schlafphasen gesteuert wird und die Schlafphasen durch Bewegungssensoren (5,6) in der Decke (1) ermittelt werden, die mit einer Aufzeichnungslogik verbunden sind, und die Beheizung der Decke (1) reduziert oder abgeschaltet wird in Tiefschlafphasen, die durch eine Bewegungslosigkeit gekennzeichnet sind.

2. Elektrisch heizbare Decke nach Anspruch 1, wobei kommende Schlafphasen durch Auswertung gespeicherter Verläufe prognostiziert werden.

3. Elektrisch heizbare Decke nach Anspruch 1, wobei erneuter Druck auf einen Start-Knopf vor Durchlaufen eines regulären Durchlaufs als Störung durch mögliche Untertemperatur registriert wird.

4. Elektrisch heizbare Decke nach einem oder mehreren der vorhergehenden Ansprüche, wobei die Abschaltung der Heizleistung auch zum Wecken eines Nutzers zu einer Weckzeit verwendet wird - oder dies zumindest unterstützt.

## Revendications

1. Plafond chauffée électriquement (1),
**caractérisé en ce que** le plafond (1) dispose d'une régulation en fonction de la profondeur de sommeil pour la réduction du poids et n'a pas d'effet d'isolation thermique appréciable, le chauffage du plafond (1) étant commandé en fonction de phases de sommeil déterminées et les phases de sommeil étant déterminées par des détecteurs de mouvement (5,6) dans le plafond (1) qui sont connectés à une logique d'enregistrement, et le chauffage du plafond est réduit ou interrompu dans les phases de sommeil profond, **caractérisées par** l'immobilité.

2. Plafond chauffée électriquement selon la revendiction 1, dans lequel les phases de sommeil à venir sont pronostiquées par l'évaluation des processus stockés.

3. Plafond chauffée électriquement selon la revendiction 1, dans lequel une nouvelle pression sur un bouton de démarrage avant de passer par un cycle régulier est enregistrée comme un défaut dû à une éventuelle sous-température.

4. Plafond chauffée électriquement selon l'une ou plusieurs des revendictions précédentes, dans lequel la désactivation de la puissance de chauffage est également utilisée pour réveiller un utilisateur à une heure de réveil - ou du moins soutenir ceci.
